# EUROPEAN PATENT SPECIFICATION

(11) **EP 3 727 177 B1**
(45) Date of publication and mention of the grant of the patent: **16.08.2023**
(21) Application number: 18830656.7
(22) Date of filing: 17.12.2018
(51) Int. Cl.: A61B 18/14, A61B 18/00, A61B 17/00

(54) **BALLOON CATHETER WITH BULBOUS SHAPED RADIOFREQUENCY (RF) ABLATION ELECTRODES**
BALLONKATHETER MIT BULBUSFÖRMIGEN HOCHFREQUENZ(HF)-ABLATIONSELEKTRODEN
CATHÉTER À BALLONNET AVEC ÉLECTRODES D'ABLATION RADIO-FRÉQUENCE (RF) EN FORME DE BULBE

(30) Priority: 19.12.2017 US 201715847661
(43) Date of publication of application: 28.10.2020
(73) Proprietor: Biosense Webster (Israel) Ltd., Yokneam 2066717 (IL)
(72) Inventor: BEECKLER, Christopher, Thomas, Irwindale, California 91706 (US); GOVARI, Assaf, 2066717 Yokneam (IL)
(74) Representative: Carpmaels & Ransford LLP
(86) International application number: PCT/US2018/065971
(87) International publication number: WO 2019/126022

(56) References cited:
- US-A1- 2015 105 774
- US-A1- 2017 296 266
- US-A1- 2017 312 024
- US-B2- 9 333 031

## Description

### FIELD OF THE INVENTION

The present invention relates generally to medical probes, and particularly to balloon catheters.

### BACKGROUND OF THE INVENTION

Various known catheter designs have an inflatable radiofrequency ablation balloon fitted at their distal end. For example, International Patent Application Publication PCT/US2014/033393 describes cardiac ablation catheters and methods of use. In some embodiments the catheter includes at least one camera inside an expandable membrane for visualizing an ablation procedure. In an embodiment, electrodes are disposed over an inflatable balloon. The electrodes are configured and positioned so as to deliver ablative radiofrequency energy to tissue when the balloon is inflated. An electrode or group of electrodes can be visually identified based on their shape. For example, groups of electrodes can be circular, oval, hexagonal, rectangular, square, etc.

As another example, U.S. Patent Application Publication 2010/0204560 describes a tissue electrode assembly that includes a membrane configured to form an expandable, conformable body that is deployable in a patient. The assembly further includes a flexible circuit positioned on a surface of the membrane and comprising at least one base substrate layer, at least one insulating layer and at least one planar conducting layer. An electrically-conductive electrode covers at least a portion of the flexible circuit and a portion of the surface of the membrane not covered by the flexible circuit, wherein the electrically-conductive electrode is foldable upon itself with the membrane to a delivery conformation having a diameter suitable for minimally-invasive delivery of the assembly to the patient. The shape and pattern of the electrodes can vary. The surface area, shape and pattern of the electrodes can influence the amount of energy applied and the ablation line created. Various electrode patterns and electrode shapes considered herein including, but not limited to, circular, rectangular, octagonal and polygonal.

US20170296266A1 provides cardiac tissue ablation catheters including an inflatable and flexible toroidal or spherically shaped balloon disposed at a distal region of an elongate member, a flexible circuit carried by an outer surface of the balloon, the flexible circuit including, a plurality of flexible branches conforming to the radially outer surface of the balloon, each of the plurality of flexible branches including a substrate, a conductive trace carried by the substrate, and an ablation electrode carried by the substrate, the ablation electrode in electrical communication with the conductive trace, and an elongate shaft comprising a guidewire lumen extending in the elongate member and extending from a proximal region of the inflatable balloon to distal region of the inflatable balloon and being disposed within the inflatable balloon, wherein a distal region of the elongate shaft is secured directly or indirectly to the distal region of the inflatable balloon.

US20150105774A1 provides medical devices and methods for making and using medical devices. An example medical device may include a catheter shaft. An expandable member may be coupled to the catheter shaft. The expandable member may be capable of shifting between a folded configuration and an expanded configuration. A plurality of flexible elements may be attached to the expandable member, with a plurality of electrode assemblies disposed on the flexible elements. The flexible elements may have a grooved substrate.

US20170312024A1 provides ablation systems and methods that control lesion depth and width such that, for example, wide and shallow lesions can be formed in target tissue in an anatomic structure of a patient during a medical procedure. Such wide and shallow lesions can be useful for treating, for example, thin tissue such as atrial tissue in atria of the heart of the patient.

US9333031B2 provides an inflatable assembly that can be positioned within a patient, including an expandable membrane, an imaging member disposed within the expandable membrane, a diffuse reflector secured relative to the expandable membrane, and a light source disposed within the expandable membrane and positioned to direct light towards the diffuse reflector such that diffuse reflection of the light is directed towards a field of view of the imaging member.

### SUMMARY OF THE INVENTION

An embodiment of the present invention provides a medical instrument including a shaft, an inflatable balloon and a Radiofrequency (RF) ablation electrode. The shaft is configured for insertion into a body of a patient. The inflatable balloon is coupled to a distal end of the shaft. The RF ablation electrode is disposed on an external surface of the balloon and has a distal edge configured to reduce electric field angular gradients of an RF electric field emitted from the distal edge.

The electrode includes an elongate main section that progressively narrows towards the distal edge, and a distal-end section, which is connected to a distal-most end of the main section and is wider than the main section at the distal-most end. In some embodiments, the distal edge of the electrode has a bulbous shape having at least a given radius of curvature. In an embodiment, by reducing the electric field angular gradients, the distal edge is configured to reduce charring deposits on the electrode.

There is additionally provided, in accordance with an embodiment of the present invention, a method for manufacturing a medical instrument, including disposing, on an external surface of an inflatable balloon, a Radiofrequency (RF) ablation electrode having a distal edge configured to reduce electric field angular gradients of an RF electric field emitted from the distal edge. The inflatable balloon is coupled to a shaft configured for insertion into a body of a patient. The electrode includes an elongate main section that progressively narrows towards the distal edge, and a distal-end section, which is connected to a distal-most end of the main section and is wider than the main section at the distal-most end.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a catheter-based ablation system comprising a Radiofrequency (RF) ablation balloon, in accordance with an embodiment of the present invention;
Fig. 2 is a schematic, pictorial illustration of an ablation balloon comprising bulbous shaped RF ablation electrodes, in accordance with an embodiment of the present invention; and
Fig. 3 is a schematic, pictorial illustration comparing electric field gradients generated at distal edges of RF ablation electrodes, in accordance with an embodiment of the present invention.

### DETAILED DESCRIPTION OF EMBODIMENTS

### OVERVIEW

Embodiments of the present invention that are described hereinafter provide an RF ablation balloon comprising electrodes designed to reduce adverse effects of an ablation, such as charring of tissue or blood due to excessive electric field gradients.

Charring of tissue or blood during ablation is a highly undesired side-effect. One problem encountered in RF ablation is tissue charring around the electrode, which produces a phenomenon called 'roll-off' (sudden increase in impedance), in which the tissue surrounding the electrode increases its electrical resistance and with it the circuit impedance. As a result, current circulation stops and no further damage to tissue is produced.

Typically, charring would also convert part of the electrode surface from a good electrical conductor to a poor one, which may cause a sharp drop in ablating electrical currents. As noted above, such drop would limit the depth of ablated tissue (also in other subsequent locations, where tissue itself in not charred but the electrode in use is at least partially covered with char), and hence reduce the efficacy of the ablation procedure. Moreover, in rare cases, charring may cause Thromboembolic complications (i.e., thrombosis of blood embolisms) that might subsequently result in a severe clinical outcome, such as a stroke.

Despite efforts to eliminate charring, for example by tissue cooling and localized blood dilution using irrigation, charring may still be evident around distal ends (i.e., distal edges) of RF ablation electrodes, where the edges of electrodes (i.e., the electrodes contour) have a small radius of curvature.

The cause is estimated in the present disclosure to be systematic: as the conducting edge of such electrode is a line of constant potential, ϕ, the electric field E = -Vcp, should be perpendicular to the electrode edge at every point along the edge. A sharply curved edge of the distal end of an electrode results in the field lines diverging at large angles relative to each other. Thus, large field gradients occur at sharp curves. When passing through tissue or blood, exceedingly large field gradients may result in charring.

In some embodiments of the present invention, an ablation balloon fitted at the distal end of a catheter comprises RF ablation electrodes having an enlarged rounded distal edge. Namely, instead of electrodes that are typically continuously narrowing distally and have a pointed distal end, the disclosed electrodes end with a bulbous, circular area, having a relatively large radius of curvature.

The disclosed RF ablation balloon, which comprises electrodes having an enlarged rounded distal edge, may reduce tissue charring that may be caused by the high electrical field gradients associated with the small radius of curvature at the electrode distal edge. By reducing tissue charring, the disclosed RF ablation balloon may reduce potentially severe clinical side effects of RF balloon ablation procedures. In addition, avoiding charring may also reduce reliability issues and increase the lifetime of the electrodes.

### SYSTEM DESCRIPTION

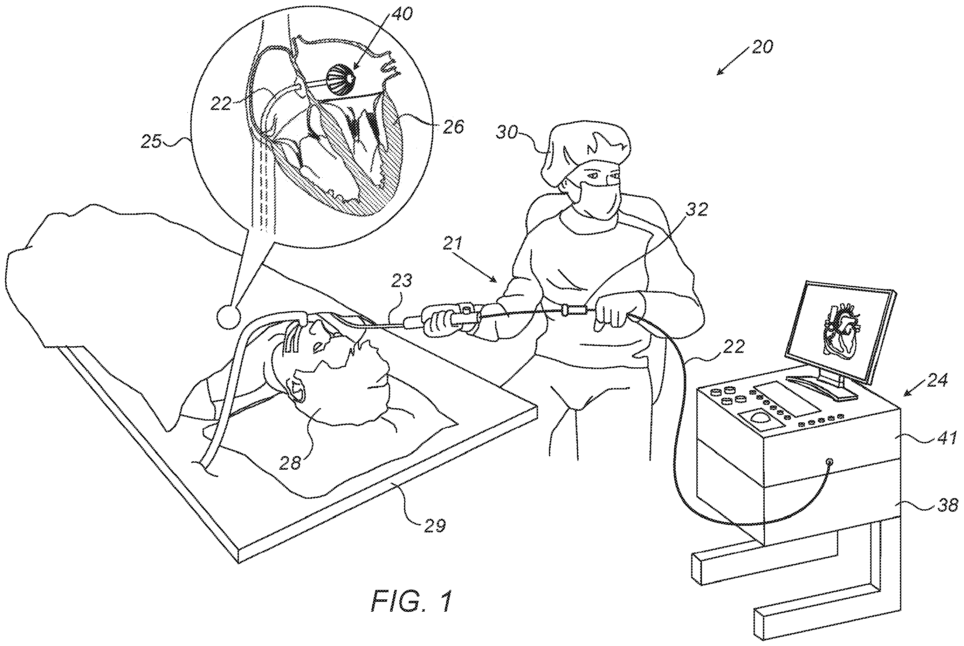

Fig. 1 is a schematic, pictorial illustration of a catheter-based ablation system 20 comprising an RF ablation balloon 40, in accordance with an embodiment of the present invention. System 20 comprises a catheter 21, wherein a distal end of shaft 22 of the catheter is inserted through a sheath 23 into a heart 26 of a patient 28 lying on a table 29. The proximal end of catheter 21 is connected to a control console 24. In the embodiment described herein, catheter 21 may be used for any suitable therapeutic and/or diagnostic purposes, such as electrical sensing and/or ablation of tissue in heart 26.

Physician 30 navigates the distal end of shaft 22 to a target location in heart 26 by manipulating shaft 22 using a manipulator 32 near the proximal end of the catheter and/or deflection from the sheath 23. During the insertion of shaft 22, balloon 40 is maintained in a collapsed configuration by sheath 23. By containing balloon 40 in a collapsed configuration, sheath 23 also serves to minimize vascular trauma along the way to target location.

Control console 24 comprises a processor 41, typically a general-purpose computer, with suitable front end and interface circuits 38 for receiving signals from catheter 21, as well as for applying treatment via catheter 21 in heart 26 and for controlling the other components of system 20. Processor 41 typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

The example configuration shown in Fig. 1 is chosen purely for the sake of conceptual clarity. The disclosed techniques may similarly be applied using other system components and settings. For example, system 20 may comprise other components and perform non-cardiac ablative treatments.

### BALLOON CATHETER WITH BULBOUS SHAPED RF ELECTRODES

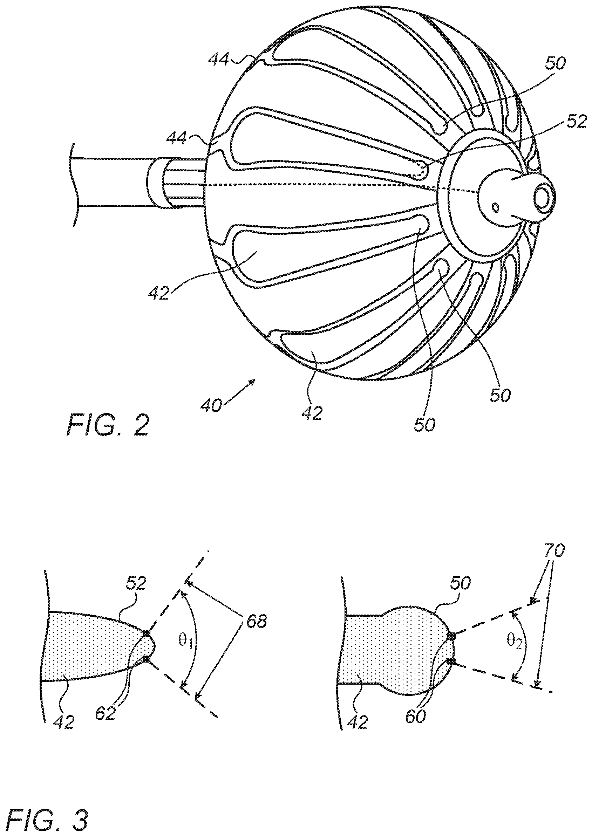

Fig. 2 is a schematic, pictorial illustration of ablation balloon 40 comprising bulbous shaped RF ablation electrodes 42, in accordance with an embodiment of the invention. As seen, electrodes 42, which are disposed over substrates 44, comprise each of a rounded edge 50, at their distal end.

As seen, rounded (i.e., bulbous) edge 50 increases the radius of curvature of the electrode at its distal end, which otherwise would tend to narrow (as dashed line 52 shows), and to potentially generate high local electrical fields. In other words, a given ablation electrode 42 comprises (i) an elongate main section that progressively narrows towards the distal edge of the electrode, and (ii) a distal-end section, which is connected to the distal-most end of the main section and is wider than the main section at the distal-most end.

The example illustration shown in Fig. 2 is chosen purely for the sake of conceptual clarity. Other designs of curvatures of the distal end are possible, for example elliptical ones, or combinations of various rounded shapes.

Fig. 3 is a schematic, pictorial illustration comparing electric field gradients generated at distal edges of RF ablation electrodes, in accordance with an embodiment of the invention. The electrode shown on the left-hand side of the figure is a hypothetical conventional electrode having a relatively pointed end. The electrode shown on the right-hand side of the figure, in accordance with an embodiment of the present invention, comprises an end having an increased radius of curvature.

The size electric field gradient between locations 62 (or between locations 60) on the edge of electrode 42, exemplified in Fig.3, is proportional to the angle between normal lines 68 (or 70) to the electrode edge at positions 62 (or 60), respectively.

As seen, angle θ₂ at the bulbous edge is smaller than corresponding angle θ₁ at a common edge. Thus, the increased curvature 50, relative to curvature 52, serves to decrease angular electrical field gradients.

The example illustration shown in Fig. 3 is chosen purely for the sake of conceptual clarity. Other designs of curvatures of the distal end are possible for lowering angular electric field gradients at a distal edge of electrode 42. One alternative example is an elliptical distal edge, or a distal edge having variable curvature so as to, for example, mitigate local excessive charring, i.e., at specific locations over the distal edge of an electrode.

Although the embodiments described herein mainly address pulmonary vein isolation, the methods and systems described herein can also be used in other applications, such as otolaryngology or neurology procedures.

It will thus be appreciated that the embodiments described above are cited by way of example, and that the present invention is not limited to what has been particularly shown and described hereinabove. Rather, the scope of the present invention is defined by the appended claims and includes both combinations and sub-combinations of the various features described hereinabove, as well as variations and modifications thereof which would occur to persons skilled in the art upon reading the foregoing description and which are not disclosed in the prior art, to the extend that said variations and modifications fall within the scope of the appended claims.

## Claims

1. A medical instrument (21), comprising:
a shaft (22) for insertion into a body of a patient (28) ;
an inflatable balloon (40) coupled to a distal end of the shaft (22); and
a Radiofrequency (RF) ablation electrode (42), which is disposed on an external surface of the balloon (40) and which has a distal edge (50) configured to reduce electric field angular gradients of an RF electric field emitted from the distal edge (50),
**characterised in that** the electrode comprises:
an elongate main section that progressively narrows towards the distal edge (50); and
a distal-end section, which is connected to a distal-most end of the main section and is wider than the main section at the distal-most end.

2. The medical instrument (21) according to claim 1, wherein the distal edge (50) of the electrode has a bulbous shape having at least a given radius of curvature.

3. The medical instrument (21) according to claim 1, wherein, by reducing the electric field angular gradients, the distal edge (50) is configured to reduce charring deposits on the electrode (42).

4. A method for manufacturing a medical instrument (21), the method comprising:
disposing, on an external surface of an inflatable balloon (40), a Radiofrequency (RF) ablation electrode (42) having a distal edge (50) configured to reduce electric field angular gradients of an RF electric field emitted from the distal edge (50); and
coupling the inflatable balloon (40) to a shaft (22) configured for insertion into a body of a patient (28), **characterised in that** the electrode comprises:
an elongate main section that progressively narrows towards the distal edge (50); and
a distal-end section, which is connected to a distal-most end of the main section and is wider than the main section at the distal-most end.

5. The method according to claim 4, wherein the distal edge (50) of the electrode (42) has a bulbous shape having at least a given radius of curvature.

## Patentansprüche

1. Medizinisches Instrument (21), umfassend:
einen Schaft (22) zum Einführen in einen Körper eines Patienten (28);
einen befüllbaren Ballon (40), der an ein distales Ende des Schafts (22) gekoppelt ist; und
eine Hochfrequenz- (HF)-Ablationselektrode (42), die auf einer Außenoberfläche des Ballons (40) angeordnet ist und einen distalen Rand (50) aufweist, der ausgestaltet ist, um Winkelgradienten des elektrischen Feldes von einem elektrischen HF-Feld zu reduzieren, das von dem distalen Rand (50) emittiert wird,
**dadurch gekennzeichnet, dass** die Elektrode umfasst:
ein längliches Hauptsegment, das progressiv zu dem distalen Rand (50) schmaler wird; und
ein distales Endsegment, das mit einem distalsten Ende des Hauptsegments verbunden ist und breiter als das Hauptsegment an dem distalsten Ende ist.

2. Medizinisches Instrument (21) nach Anspruch 1, wobei der distale Rand (50) der Elektrode eine Bulbusform mit mindestens einem gegebenen Krümmungsradius hat.

3. Medizinisches Instrument (21) nach Anspruch 1, wobei der distale Rand (50) ausgestaltet ist, um durch Reduzieren der Winkelgradienten des elektrischen Felds Verkohlungsablagerungen auf der Elektrode (42) zu reduzieren.

4. Verfahren zum Fertigen eines medizinischen Instruments (21), wobei das Verfahren umfasst:
Anordnen einer Hochfrequenz- (HF)-Ablationselektrode (42) mit einem distalen Rand (50), der ausgestaltet ist, um Winkelgradienten des elektrischen Feldes von einem elektrischen HF-Feld, das von dem distalen Rand (50) emittiert wird, zu reduzieren, auf einer Außenoberfläche eines befüllbaren Ballons (40); und
Koppeln des befüllbaren Ballons (40) an einen Schaft (22), der zum Einführen in einen Körper eines Patienten (28) ausgestaltet ist, **dadurch gekennzeichnet, dass** die Elektrode umfasst:
ein längliches Hauptsegment, das progressiv zu dem distalen Rand (50) schmaler wird; und
ein distales Endsegment, das mit einem distalsten Ende des Hauptsegments verbunden ist und breiter als das Hauptsegment an dem distalsten Ende ist.

5. Verfahren nach Anspruch 4, wobei der distale Rand (50) der Elektrode (42) eine Bulbusform mit mindestens einem gegebenen Krümmungsradius hat.

## Revendications

1. Instrument médical (21) comprenant :
une tige (22) destinée à être insérée dans le corps d'un patient (28) ;
un ballonnet gonflable (40) couplé à une extrémité distale de la tige (22) ; et
une électrode d'ablation radiofréquence (RF) (42), disposée sur une surface externe du ballonnet (40) et dotée d'un bord distal (50) configuré pour réduire les gradients angulaires de champ électrique d'un champ électrique RF émis par le bord distal (50),
**caractérisée en ce que** l'électrode comprend :
une section principale allongée qui se rétrécit progressivement vers le bord distal (50) ; et
une section d'extrémité distale, qui est reliée à une extrémité la plus distale de la section principale et qui est plus large que la section principale à l'extrémité la plus distale.

2. Instrument médical (21) selon la revendication 1, le bord distal (50) de l'électrode ayant une forme bulbeuse ayant au moins un rayon de courbure donné.

3. Instrument médical (21) selon la revendication 1, en réduisant les gradients angulaires du champ électrique, le bord distal (50) étant configuré pour réduire les dépôts de carbonisation sur l'électrode (42).

4. Procédé de fabrication d'un instrument médical (21), le procédé comprenant :
la disposition, sur une surface externe d'un ballonnet gonflable (40), d'une électrode d'ablation radiofréquence (RF) (42) ayant un bord distal (50) configuré pour réduire les gradients angulaires de champ électrique d'un champ électrique RF émis à partir du bord distal (50) ; et
le couplage du ballonnet gonflable (40) à une tige (22) configurée pour être insérée dans le corps d'un patient (28), **caractérisé en ce que** l'électrode comprend :
une section principale allongée qui se rétrécit progressivement vers le bord distal (50) ; et
une section d'extrémité distale, reliée à une extrémité la plus distale de la section principale et plus large que la section principale à l'extrémité la plus distale.

5. Procédé selon la revendication 4, le bord distal (50) de l'électrode (42) ayant une forme bulbeuse ayant au moins un rayon de courbure donné.
